# EUROPEAN PATENT SPECIFICATION

(11) **EP 4 736 797 B1**
(45) Date of publication and mention of the grant of the patent: **19.08.2026**
(21) Application number: 25210882.4
(22) Date of filing: 23.10.2025
(51) Int. Cl.: A61B 18/14, A61B 10/02

(54) **FULLY AUTOMATIC BIOPSY NEEDLE WITH HEMOSTASIS FUNCTION**
VOLLAUTOMATISCHE BIOPSIENADEL MIT HÄMOSTASEFUNKTION
AIGUILLE DE BIOPSIE ENTIÈREMENT AUTOMATIQUE À FONCTION D'HÉMOSTASE

(30) Priority: 30.10.2024 CN 202411525606; 25.09.2025 CN 202522069702 U
(43) Date of publication of application: 06.05.2026
(73) Proprietor: CURAWAY MEDICAL TECHNOLOGY CO., LTD., Hangzhou Zhejiang Province 310018 (CN)
(72) Inventor: CHEN, Qiang, Hangzhou, 310018 (CN); ZHANG, Yao, Hangzhou, 310018 (CN); WANG, Zhiqing, Hangzhou, 310018 (CN)
(74) Representative: Argyma

(56) References cited:
- EP-B1- 1 440 665
- WO-A1-2009/156506
- CN-A- 115 624 355
- US-A1- 2005 234 443

## Description

### TECHNICAL FIELD

The invention relates to the technical field of medical device, particularly to a fully automatic biopsy needle with hemostasis function and a biopsy needle.

### BACKGROUND ART

Biopsy is an important means for tumor diagnosis. With an increasing incidence of tumors, the demand for biopsy procedures has been growing annually. According to statistics, the global market size for biopsy needles reached billions of dollars in 2020 and is expected to maintain rapid growth in the coming years. Biopsy procedures are often accompanied by bleeding, necessitating the use of products with hemostasis functions to reduce bleeding risks. With the growth of the biopsy market, the invention also possesses significant technical advantages and market prospects.

At present, there are some ablation needles with hemostasis functions on the market, but they often have the following shortcomings:
Bulky size and complex operation: They require connection to external radiofrequency or microwave hosts, which increases the difficulty and risks of surgical procedures.
High cost: Radiofrequency or microwave hosts are expensive, leading to increased surgical costs.
Inadequate hemostatic effect: Some products have ablation-based hemostatic functions but cannot precisely target the biopsy needle tract. This results in poor hemostasis or may cause secondary puncture injuries or bleeding (e.g., the high-temperature carbonization and adhesion of the needle tip can tear other tissues during needle withdrawal, leading to secondary bleeding).
For example, EP1440665 B1 relates to apparatus for therapeutic cauterization of predetermined volumes of biological tissue according to prior art.

### Summary of the Invention

An objective of the invention is to provide a biopsy needle with hemostasis function and a biopsy needle that can promptly perform hemostasis on the needle tract after tissue biopsy. It features simple operation, low cost, and high safety, eliminating the need for expensive emergency medical resources. This provides precise diagnosis and treatment for tumor patients with high bleeding risks or coagulation disorders, while avoiding excessive consumption of emergency medical resources and promoting the efficient utilization of healthcare resources.

The technical solution provided by the invention is: a fully automatic biopsy needle with hemostasis function, which includes:
a sampling needle having a sampling groove provided at a distal end thereof;
a cutting needle assembly including a cutting needle and an inner insulating layer covering a periphery of the cutting needle, where a cutting edge that matches the sampling groove is provided at a distal end of the cutting needle, the cutting needle is fitted over a periphery of the sampling needle, with the distal end of the cutting needle exposed beyond the inner insulating layer to form a first electrode with the distal end of the sampling needle; the cutting needle includes a cutting part at the distal end and a conveying part connected to a proximal end of the cutting part, with an outer diameter of the conveying part being smaller than that of the cutting part, and the inner insulating layer covers the conveying part;
a cannula assembly including a cannula, an outer insulating layer, and a cannula plug, where the outer insulating layer covers a periphery of a cannula, with a distal end of the cannula exposed beyond the outer insulating layer to form a second electrode, the cannula assembly is configured to be detachably connected to a handle of the biopsy needle via the cannula plug, allowing the cannula to be coaxially fitted over the periphery of the cutting needle and detachable; and
the proximal ends of the sampling needle and the cannula plug are respectively electrically connected to a power supply device, the cannula assembly being configured to be disassembled from the handle after biopsy sampling, leaving the cannula assembly in a needle tract, the sampling needle and the cutting needle assembly being configured to be inserted into the cannula after removing a sample tissue from the sampling groove, the power supply device being configured to apply electrical energy to perform hemostasis operation on the needle tract using the first electrode and the second electrode after the the sampling needle and the cutting needle assembly have been reinserted into the cannula.

Preferably, the biopsy needle further includes a distance adjusting block, which is slidably connected to the handle, with a distal end of the distance adjusting block fixedly connected to the cannula plug, after inserting the sampling needle and the cutting needle assembly into the cannula, the biopsy needle is configured to at least include a sampling configuration and a hemostasis configuration;
in the sampling configuration, when the sampling needle and the cutting needle complete sampling, a proximal end of the sampling groove extends beyond the distal end of the cannula; and
in the hemostasis configuration, when the sampling needle and the cutting needle maintain the completed sampling, the distance adjusting block and the cannula assembly are adjusted a preset distance distally along a length of a needle body.

Preferably, a cross-section of the distance adjusting block matches that of the handle, with sliding buckles provided on two sides of an inner wall of the distance adjusting block extending along the length, an outer surface of the handle is provided with chutes that match the sliding buckles, the distance adjusting block is configured to be slidably connected to the handle via the sliding buckles and the chutes to enable distance adjustment along the length, after adjusting the distance adjusting block to a predetermined position, a position of the distance adjusting block is locked by a locking structure.

Preferably, the locking structure includes a plurality of locking holes penetrating a surface of the distance adjusting block and an elastic arm locking button with one end fixedly arranged on the handle, a protrusion on a free end of the elastic arm locking button is engaged with one of the locking holes to lock axial relative positions of the first electrode and the second electrode.

Preferably, the biopsy needle further includes an energy transmission socket, which is fixedly connected to the proximal end of the handle, with the distal end being a free end, an elastic arm locking button for locking the axial relative positions of the first electrode and the second electrode is arranged on the free end of the energy transmission socket, and two electrical connection ends on the energy transmission socket are respectively electrically connected to the cannula plug and the proximal end of the sampling needle.

Preferably, a first wiring groove is provided at the inner wall of the distance adjusting block, and a second wiring groove connected to the first wiring groove is provided at an inner wall of the energy transmission socket, conducting wires connecting the cannula plug to the energy transmission socket and the sampling needle to the energy transmission socket extend along the first and/or second wiring grooves.

Preferably, the present application further includes a portable power supply device, which is electrically connected to the energy transmission socket through an electrical pin, where the portable power supply device includes a lithium battery, a DCDC module, an MCU controller, an H-bridge module, and a step-up transformer, the lithium battery supplies power to the MCU controller through the DCDC module, the lithium battery is electrically connected to the H-bridge module, and the MCU controller outputs a driving signal to drive the H-bridge module to output current to the first electrode and the second electrode through the step-up transformer for tissue hemostasis.

Preferably, the portable power supply device is detachably connected to the proximal end of the handle, a first magnetic attraction piece is provided inside a distal end of the portable power supply device, and a second magnetic attraction piece is provided inside the proximal end of the handle, the distal end of the portable power supply device is provided with elastic arms with convex clamping points on two sides of a circumference, and the proximal end of the handle is provided with concave clamping points that match the convex clamping points.

Preferably, the biopsy needle is configured to detect impedance in real-time via a control system during hemostasis operation, when the detected impedance meets a preset condition, the biopsy needle is retracted a preset distance and the hemostasis operation is repeated until the biopsy needle completely exits the needle tract.

Based on the same concept, the invention also provides a biopsy needle with hemostasis function, which includes:
a sampling needle having a sampling groove provided at a distal end thereof;
a cutting needle assembly including a cutting needle and an inner insulating layer covering a periphery of the cutting needle, where a cutting edge that matches the sampling groove is provided at a distal end of the cutting needle, the cutting needle is fitted over a periphery of the sampling needle, with the distal end of the cutting needle exposed beyond the inner insulating layer to form a first electrode with the distal end of the sampling needle;
a cannula assembly including a cannula, an outer insulating layer, and a cannula plug, where the outer insulating layer covers a periphery of a cannula, with a distal end of the cannula exposed beyond the outer insulating layer to form a second electrode, the cannula assembly is configured to be detachably connected to a handle of the biopsy needle via the cannula plug, allowing the cannula to be coaxially fitted over the periphery of the cutting needle and detachable; and
the proximal ends of the sampling needle and the cannula plug are respectively electrically connected to a power supply device, the operators disassemble the cannula assembly from the handle after biopsy sampling, leaving the cannula assembly in a needle tract, and then insert the sampling needle and the cutting needle assembly into the cannula after removing a sample tissue from the sampling groove, then the power supply device applies electrical energy to perform hemostasis operation on the needle tract using the first electrode and the second electrode.

Compared with the prior art, the invention has the following advantages:
1. According to the technical solution of the present embodiment, a cannula assembly is provided on the one hand to retain the needle tract and on the other hand to form two electrodes with the sampling needle and the cutting needle for needle tract ablation hemostasis. Specifically, after the sampling needle and the cutting needle complete sampling, they are withdrawn, leaving the cannula assembly in the needle tract to retain it. After removing the effectively sampled tissue, the sampling needle and the cutting needle are reinserted into the cannula, easily replicating the punctured needle tract during sampling. Moreover, the distal end of the cutting needle forms a first electrode with the distal end of the sampling needle, and the distal end of the cannula forms a second electrode. By applying appropriate current to the first electrode and the second electrode, tissue ablation and coagulation can be achieved, preventing needle tract bleeding. Furthermore, while achieving hemostasis, the sampling effect is not compromised by the presence of an insulating layer.
2. The present invention further provides a distance adjusting block. On one hand, the distance adjusting block is detachably connected to the cannula assembly to realize that the cannula assembly is left in the needle tract after sampling. On the other hand, the distance adjusting block is slidably connected to the biopsy needle handle. Since a relatively long sampling groove is provided at the distal end of the sampling needle of the biopsy needle, after sampling is completed, the distal end of the sampling needle and the cutting needle is at a relatively long distance from the distal end of the cannula, and it is necessary to perform hemostatic ablation on the site (such as a tumor) where the sample tissue is obtained. Therefore, in the present embodiment, the distance adjusting block is slidably connected to the handle. In the hemostasis configuration, the sampling needle and the cutting needle maintain the state when sampling is completed and are inserted through the cannula to return to the original needle tract for sampling. At this time, the cannula assembly is adjusted to the distal end by a preset distance through the distance adjusting block, which can, on one hand, achieve effective ablation of the site where the sample tissue is obtained, and on the other hand, reduce the distance between the first electrode and the second electrode, which is beneficial for achieving precise ablation and coagulation and avoiding excessive damage to the tissue or incomplete hemostasis.

### Brief Description of the Drawings

FIG. 1 is an overall schematic structural diagram illustrating a fully automatic biopsy needle with hemostasis function according to the present invention;
FIG. 2 is a cross-sectional view of a fully automatic biopsy needle with hemostasis function according to the present invention;
FIG. 3 is a schematic structural diagram illustrating a fixed slider for a sampling needle and a fixed slider for a cutting needle according to the present invention;
FIG. 4 is a schematic structural diagram illustrating a left guide shell and a firing switch according to the present invention;
FIG. 5 is a schematic diagram of a fully automatic biopsy needle with hemostasis function according to the present invention (with part of a shell of a handle omitted);
FIG. 6 is a schematic structural diagram illustrating a No. 1 chambering button and a No. 2 chambering button according to the present invention;
FIG. 7 is a schematic diagram of a fully automatic biopsy needle with hemostasis function according to the present invention (with part of a shell of a handle omitted);
FIG. 8 is a schematic diagram illustrating a variation in one chambering method according to the present invention;
FIG. 9 is a schematic diagram illustrating a variation in another chambering method according to the present invention;
FIG. 10 is a schematic diagram illustrating a process of firing and sampling with a fully automatic biopsy needle with hemostasis function according to the present invention;
FIG. 11 is an overall schematic diagram illustrating a fully automatic biopsy needle with hemostasis function according to the present invention (with a cannula and a distance adjusting block translated);
FIG. 12 is a schematic diagram illustrating a distance adjusting block and an energy transmission socket according to the present invention;
FIG. 13 is a schematic diagram illustrating a state where biopsy sampling is completed according to the present invention;
FIG. 14 is a schematic diagram illustrating a state where ablation hemostasis is performed according to the present invention;
FIG. 15 is a schematic diagram illustrating an internal module composition of a portable power supply device according to the present invention; and
FIG. 16 is a schematic diagram illustrating a distal end structure of a cutting needle according to one embodiment of the present invention.

### Description of reference numerals:

001-First electrode; 002-Second electrode; 1-Sampling needle; 1001-Sampling groove; 2-Cutting needle; 201-Cutting part; 202-Conveying part; 3-Inner insulating layer; 4-Cannula; 5-Outer insulating layer; 6-Cannula plug; 7-Handle; 71-Chute; 72-Second magnetic attraction piece; 8-Distance adjusting block; 81-Sliding buckle; 82-First wiring groove; 83-Locking hole; 84-Cannula socket; 9-Energy transmission socket; 91-Second wiring groove; 92-Elastic arm locking button; 10-Power supply device; 101-First magnetic attraction piece; 11-No. 1 chambering button; 111-First connection hole; 112-First guiding piece; 12-No. 2 chambering button; 121-Second guiding piece; 13-Fixed slider for sampling needle; 131-Second elastic clamping hook; 132-Second firing groove; 133-Elastic gasket; 14-Fixed slider for cutting needle; 141-First elastic clamping hook; 15-Left guide shell; 151-Elastic piece limiting groove; 152-Guiding limiting piece groove; 153-Lower slot; 16-Right guide shell; 17-First partition; 171-First clamping groove; 18-First spring; 19-Second partition; 191-Second clamping groove; 20-Second spring; 21-Firing switch; 211-Elastic piece; 212-Guiding limiting piece; 213-Linkage rod; 214-First firing groove.

### Detailed Description of the Invention

The implementation methods of the present invention are illustrated through specific examples below. A person skilled in the art can easily understand other advantages and effects of the present invention from the content disclosed in this specification. The present invention can also be implemented or applied through other different specific implementation methods. Various details in this specification can also be modified or changed based on different perspectives and applications without deviating from the spirit of the present invention. It should be understood that the terms "center", "longitudinal", "lateral", "upper", "lower", "front", "rear", "left", "right", "vertical", "horizontal", "top", "bottom", "inner", "outer", and other terms indicating orientation or positional relationships are based on the orientation or positional relationships shown in the accompanying drawings. These terms are used only to facilitate the description of the present invention and simplify the description, rather than indicating or implying that the device or element referred to must have a specific orientation or be constructed and operated in a specific orientation. Therefore, these terms should not be construed as limiting the present invention. Further, the terms "first" and "second", etc. are used for descriptive purposes only and are not to be construed as indicating or implying relative importance or implicitly indicating the number of technical features indicated. Thus, features defined with "first" or "second" may explicitly or implicitly include one or more of such features. In the description of the present invention, unless otherwise specified, the meaning of "a plurality of' is two or more.

It should be noted that unless explicitly stated or defined otherwise, terms such as "mounted", "connected to" and "connected" should be interpreted broadly. For example, a connection may be fixed or detachable, or integral; it may be a mechanical connection, a power-connecting, or a direct connection, or it may be indirectly connected through an intermediate medium. It may also refer to internal communication between two components. The specific meaning of the above terms in the present invention can be understood by a person skilled in the art according to specific circumstances.

Biopsy is the gold standard for tumor pathology diagnosis and is widely used in clinical applications. Traditional biopsy needles obtain tissue samples through mechanical cutting, but this method can easily lead to bleeding, especially for biopsies of special sites such as the liver and kidneys, where the bleeding risk is higher and may even endanger the patient's life.

Biopsies include: bone biopsy, tissue biopsy, and cell biopsy. For solid tumors, tissue biopsy is generally used to diagnose pathological tissues. Solid tumors include the liver, lungs, kidneys, prostate, thyroid, and so on. The principle of tissue biopsy generally involves using spring force to achieve rapid cutting of pathological tissues. A typical tissue biopsy needle consists of a needle core and a needle tube. The needle core is generally equipped with a sampling groove 1001, and the needle tube is mainly used to achieve rapid cutting of tissues, thereby retaining the tissues in the sampling groove 1001. Biopsy needles come in various sizes, with large ones including 7G, 9G, and 12G, and small ones including 16G, 18G, and 20G, among others. Due to the mechanical cutting and puncturing operations, bleeding has become the most frequent complication during the biopsy process. Of course, in general cases, this bleeding can be easily coagulated (hemostasis). However, in some special organs, special sites, or for some special patients, such as those with coagulation disorders, massive bleeding can easily occur. In such scenarios, biopsies cannot be normally conducted. Of course, there are some clinical methods for analyzing such bleeding, such as using radiofrequency or microwave for needle tract hemostasis. However, these cases belong to emergency medical treatment, which is costly and operationally complex.

### Embodiment 1

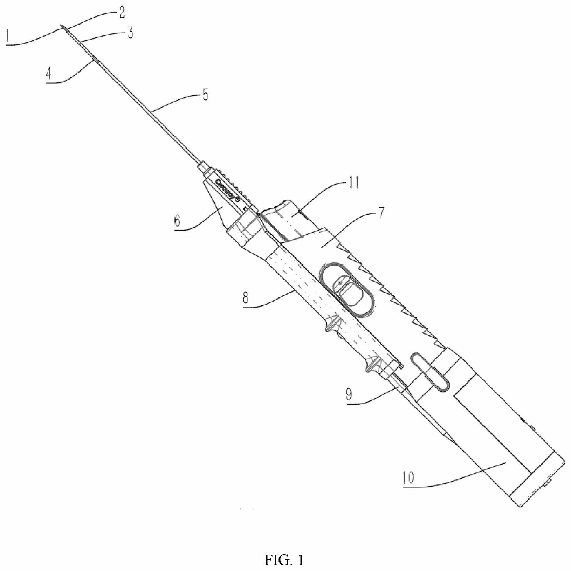

To address this, the present embodiment provides a fully automatic biopsy needle with hemostasis function, as shown in FIG. 1, which includes:
a sampling needle 1 having a sampling groove 1001 provided at a distal end thereof;
a cutting needle assembly including a cutting needle 2 and an inner insulating layer 3 covering a periphery of the cutting needle 2, where a cutting edge that matches the sampling groove 1001 is provided at a distal end of the cutting needle 2, the cutting needle 2 is fitted over the periphery of the sampling needle 1, with the distal end of the cutting needle 2 is exposed from the inner insulating layer 3 to form a first electrode 001 with the distal end of the sampling needle 1. The cutting needle 2 includes a cutting part 201 at the distal end thereof and a conveying part 202 connected to a proximal end of the cutting part, with an outer diameter of the conveying part 202 being smaller than that of the cutting part 201, and the inner insulating layer 3 covers the conveying part 202;
a cannula assembly including a cannula 4, an outer insulating layer 5, and a cannula plug 6, where the outer insulating layer 5 covers a periphery of a cannula 4, with a distal end of the cannula 4 exposed beyond the outer insulating layer 5 to form a second electrode 002, the cannula assembly is configured to be detachably connected to a handle 7 of a biopsy needle via the cannula plug 6, allowing the cannula 4 to be coaxially fitted over the periphery of the cutting needle 2 and be detachable;
the proximal ends of the sampling needle 1 and the cannula plug 6 are respectively electrically connected to a power supply device 10 to disassemble the cannula assembly from the handle 7 after biopsy sampling, leaving the cannula assembly in a needle tract, and then insert the sampling needle 1 and the cutting needle assembly into the cannula 4 after removing a sample tissue from the sampling groove 1001, and apply electrical energy to perform hemostasis operation on the needle tract using the first electrode 001 and the second electrode 002.

According to the technical solution of the present embodiment, a cannula assembly is provided on the one hand to retain the needle tract and on the other hand to form two electrodes with the sampling needle 1 and the cutting needle 2 for needle tract ablation hemostasis. Specifically, after the sampling needle 1 and the cutting needle 2 complete sampling, they are withdrawn, leaving the cannula assembly in the needle tract to retain it. After removing the effectively sampled tissue, the sampling needle 1 and the cutting needle 2 are reinserted into the cannula 4, easily replicating the punctured needle tract during sampling. Moreover, the distal end of the cutting needle 2 forms a first electrode 001 with the distal end of the sampling needle 1, and the distal end of the cannula 4 forms a second electrode 002. By applying appropriate current to the first electrode 001 and the second electrode 002, tissue ablation and coagulation can be achieved, preventing needle tract bleeding.

In the present embodiment, the cutting needle 2 includes a cutting part 201 at a distal end thereof and a conveying part 202 connected to the proximal end of the cutting part 201. An outer diameter of the conveying part 202 is smaller than that of the cutting part, and an inner insulating layer 3 covers the periphery of the conveying part 202, with the overall outer diameter after coverage being equal to or slightly smaller than that of the cutting part 201.

The table below shows a comparison of sampling data using the technical solution of the present embodiment:

| Sample state | Sampling weight (g) | Sampling length (mm) |
|---|---|---|
| Without insulating layer | 0.0069 | 17 |
| | 0.0083 | 17 |
| | 0.0076 | 16 |
| | 0.0072 | 17 |
| | 0.0077 | 16 |
| | 0.0070 | 16 |
| Sampling effect (with an insulating layer, outer diameters of cutting part and conveying part being equal) | 0.0064 | 15 |
| | 0.0066 | 15 |
| | 0.0057 | 14 |
| | 0.0059 | 13 |
| | 0.0057 | 13 |
| | 0.0062 | 14 |
| Sampling effect (with an insulating layer, an outer diameter of the conveying part being smaller than that of the cutting part) | 0.0072 | 17 |
| | 0.0082 | 17 |
| | 0.0075 | 16 |
| | 0.0071 | 17 |
| | 0.0068 | 16 |
| | 0.0080 | 17 |

The above data indicates that when there is no insulating layer and when there is an insulating layer with the covered insulating layer flush with the working exposed end, the sampling effects are very close and effectively equivalent. However, when there is an insulating layer with the covered insulating layer larger than the working exposed end, the sampling effect is uneven and relatively poor, with relatively higher puncturing resistance. The inner insulating layer 3 is made of a polymer material (such as Teflon, FEP, PET, etc.). It can be coated onto the cutting needle 2 by melting powdered insulating material at high temperatures (such as Teflon, etc.) or can be a thermoplastic polymer continuous tubular structural material (such as FEP, PET, etc.) that is heat-shrunk and fitted onto the cutting needle 2.

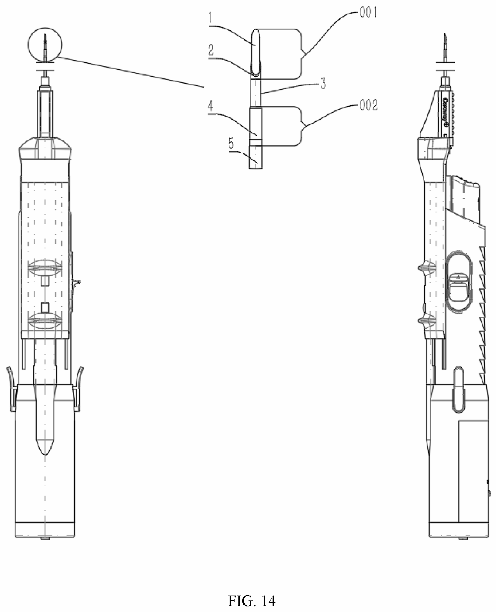

Preferably, as shown in FIGS. 1, 11, 12, 13, and 14, the biopsy needle further includes a distance adjusting block 8, which is slidably connected to the handle 7, with a distal end of the distance adjusting block 8 fixedly connected to the cannula plug 6, after inserting the sampling needle 1 and the cutting needle assembly are inserted into the cannula 4, the biopsy needle is configured to at least include a sampling configuration and a hemostasis configuration;
in the sampling configuration, when the sampling needle 1 and the cutting needle 2 complete sampling, the proximal end of the sampling groove 1001 extends beyond the distal end of the cannula 4; and
in the hemostasis configuration, when the sampling needle 1 and the cutting needle 2 maintain the completed sampling, the distance adjusting block 8 and the cannula assembly are adjusted a preset distance distally along a length of a needle body.

As a further preferred solution, the technical solution of the present embodiment provides a distance adjusting block 8. The distance adjusting block 8, on the one hand, is detachably connected to the cannula assembly (with a cannula socket provided at the distal end of the distance adjusting block to cooperate with the cannula plug for structural and electrical connection), is used to realize that the cannula assembly is left in the needle tract after sampling. On the other hand, the distance adjusting block 8 is slidably connected to the biopsy needle handle 7. Since a relatively long sampling groove 1001 is provided at the distal end of the sampling needle 1 of the biopsy needle, after sampling is completed, the distal end of the sampling needle 1 and the cutting needle 2 is at a relatively long distance from the distal end of the cannula 4, and it is necessary to perform hemostatic ablation on the site (such as a tumor) where the sample tissue is obtained. Therefore, in the present embodiment, the distance adjusting block 8 is slidably connected to the handle 7. In the hemostasis configuration, the sampling needle 1 and the cutting needle 2 maintain the state when sampling is completed and are inserted through the cannula 4 to return to the original needle tract for sampling. At this time, the cannula assembly is adjusted to the distal end by a preset distance through the distance adjusting block 8, which can, on one hand, achieve effective ablation of the site where the sample tissue is obtained, and on the other hand, reduce the distance between the first electrode 001 and the second electrode 002, which is beneficial for achieving precise ablation and coagulation and avoiding excessive damage to the tissue or incomplete hemostasis.

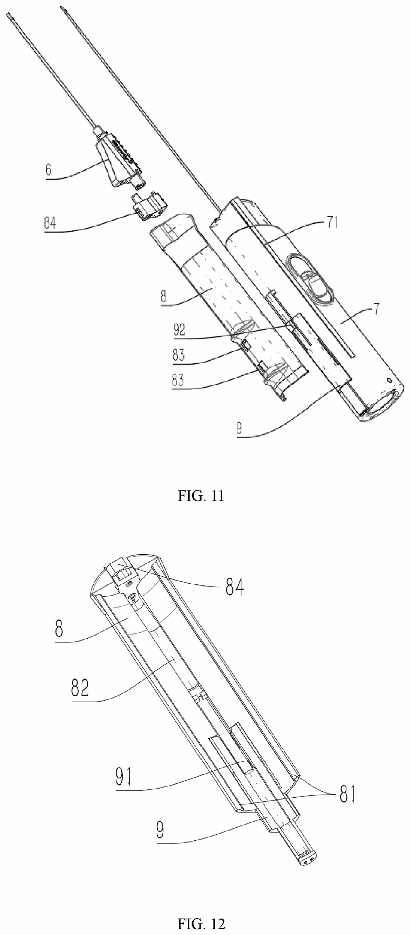

Preferably, with continued reference to FIGS. 11 and 12, a cross-section of the distance adjusting block 8 matches that of the handle 7, with sliding buckles 81 provided on two sides of an inner wall of the distance adjusting block 8 extending along the length, an outer surface of the handle 7 is provided with chutes 71 that match the sliding buckles 81, the distance adjusting block 8 is configured to be slidably connected to the handle 7 via the sliding buckles 81 and the chutes 71 to enable distance adjustment along the length, after adjusting the distance adjusting block 8 to a predetermined position, a position of the distance adjusting block 8 is locked by a locking structure.

The technical solution of the present embodiment discloses a connection structure between the distance adjusting block 8 and the handle 7. The distance adjusting block 8 is equipped with sliding buckles 81 extending lengthwise, and the outer surface of the handle 7 is provided with the chutes 71 that cooperate with the sliding buckles 81. Using this adjustment mechanism allows for a more compact structure, preventing the radial size of the operating handle 7 from becoming excessively large, making it more portable and easier to operate. However, the present invention is not limited to this single type of axially sliding adjustment structure. Any mechanism from existing technology that enables axial adjustment between two components can be used. For example, the positions of the sliding buckles 81 can be interchanged with those of the chutes 71. Preferably, with continued reference to FIG. 11, the locking structure includes a plurality of locking holes 83 penetrating a surface of the distance adjusting block 8 and an elastic arm locking button 92 with one end fixedly arranged on the handle 7, a protrusion on a free end of the elastic arm locking button 92 is engaged with one of the locking holes 83 to lock axial relative positions of the first electrode 001 and the second electrode 002.

The present embodiment provides a locking structure with a plurality of locking holes 83 provided on the surface of the distance adjusting block 8. The plurality of locking holes 83 offer various adjustment positions. The protrusion on the free end of the elastic arm locking button 92, which is fixedly provided on the handle 7, engages with one of the locking holes 83 to achieve adjustment and fixation at different positions, thereby enabling adjustment of the distance between the first electrode 001 and the second electrode 002 and providing flexibility for the coagulation operation. Of course, at least two locking holes 83 should be set, corresponding to the sampling configuration and hemostasis configuration respectively. The locking structure in the present embodiment can achieve a smaller radial dimension, facilitating operation.

Preferably, with continued reference to FIG. 11, the biopsy needle further includes an energy transmission socket 9, which is fixedly connected to the proximal end of the handle 7, with the distal end being a free end, an elastic arm locking button 92 for locking the axial relative positions of the first electrode 001 and the second electrode 002 is arranged on the free end of the energy transmission socket 9, and two electrical connection ends on the energy transmission socket 9 are respectively electrically connected to the cannula plug 6 and the proximal end of the sampling needle 1.

The technical solution of the present embodiment provides a simple and convenient connection method by providing the energy transmission socket 9, where the two electrical connection ends on the energy transmission socket 9 are electrically connected to the cannula plug 6 and the proximal end of the sampling needle 1, respectively. Additionally, the energy transmission socket 9 is electrically connected to the power supply device 10 through pins, facilitating componentization, modularization, and standardized production. More preferably, configuring the distal end of the energy transmission socket 9 as a free end allows for the placement of an elastic arm locking button 92 on the free end, which enables the energy transmission socket 9 to simultaneously perform energy transmission and mode adjustment (sampling/hemostasis) functions, further saving radial structural space in the operating handle 7 and facilitating product portability.

Preferably, with continued reference to FIG. 12, the inner wall of the distance adjusting block 8 is provided with a first wiring groove 82, and a second wiring groove 91 connected to the first wiring groove 82 is provided at an inner wall of the energy transmission socket 9, conducting wires connecting the cannula plug 6 to the energy transmission socket 9 and the sampling needle 1 to the energy transmission socket 9 extend along the first and/or second wiring grooves 82, 91. The technical solution of the present embodiment provides accommodation space for the electrically connected conducting wires by providing a first wiring groove 82 on the inner wall of the distance adjusting block 8 and a second wiring groove 91 inside the energy transmission socket 9. The wiring grooves are highly compatible with the conducting wires, preventing jamming during adjustment.

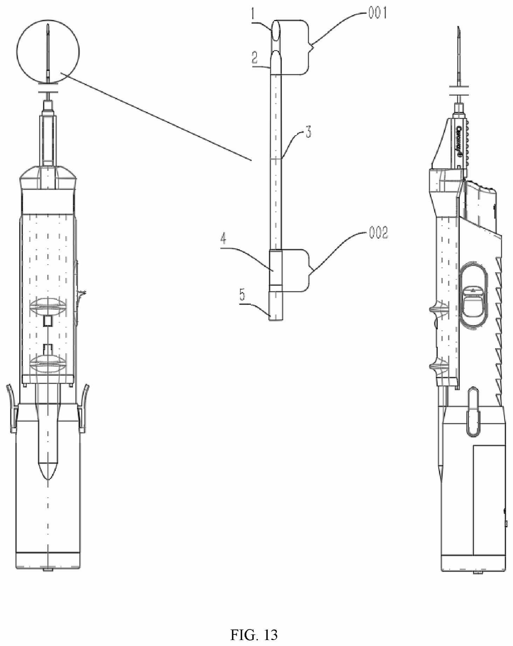

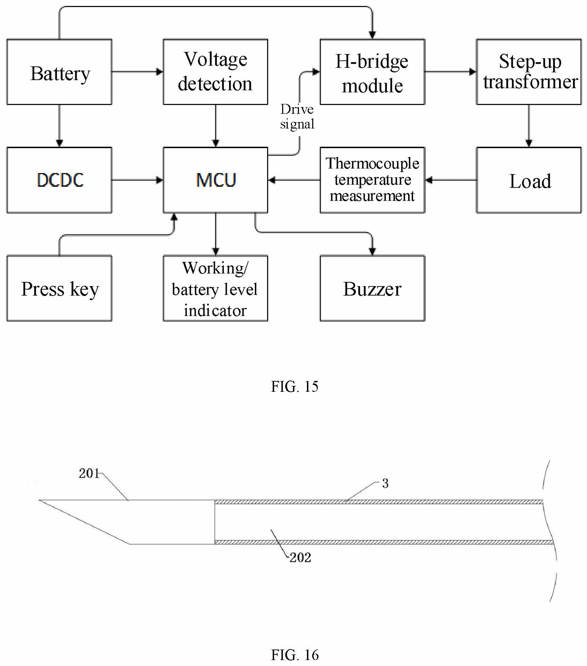

Preferably, with continued reference to FIGS. 13, 14, and 15, the present application further includes a portable power supply device 10, which is electrically connected to the energy transmission socket 9 through an electrical pin, where the portable power supply device 10 includes a lithium battery, a DCDC module, an MCU controller, an H-bridge module, and a step-up transformer, the lithium battery supplies power to the MCU controller through the DCDC module, the lithium battery is electrically connected to the H-bridge module, and the MCU controller outputs a driving signal to drive the H-bridge module to output current to the first electrode 001 and the second electrode 002 through the step-up transformer for tissue hemostasis. The technical solution of the present embodiment discloses a portable power supply device 10. In prior art, when biopsy bleeding occurs, emergency medical resources are often required, and an external radiofrequency ablation or microwave energy host is needed to provide energy for ablation hemostasis. However, the portable power supply device 10 provided in the present embodiment is only used for needle tract ablation and coagulation and does not need to reach the energy level required for tissue ablation to necrosis. Therefore, the portable power supply device 10 in the present embodiment can effectively cooperate with the first electrode 001 and the second electrode 002 for tissue ablation and coagulation operations. Referring to FIG. 15, an internal module composition diagram of the portable power supply device 10 is shown.

Preferably, the portable power supply device 10 is detachably connected to the proximal end of the handle 7, a first magnetic attraction piece 101 is provided inside a distal end of the portable power supply device 10, and a second magnetic attraction piece 72 is provided inside the proximal end of the handle 7, the distal end of the portable power supply device 10 is provided with elastic arms with convex clamping points on two sides of a circumference, and the proximal end of the handle 7 is provided with concave clamping points that match the convex clamping points.

In the present embodiment, the portable power supply device 10 adopts a modular and detachable form, providing a convenient and fast connection structure to achieve rapid connection of the portable power supply device 10. Of course, this is just one of the connection methods. The main inventive concept of the present invention does not limit the connection structure to only this one. Any fast-disassembly structure in the prior art can be used.

Preferably, the biopsy needle is configured to detect impedance in real-time via a control system during hemostasis operation, when the detected impedance meets a preset condition, the biopsy needle is retracted a preset distance and the hemostasis operation is repeated until the biopsy needle completely exits the needle tract.

The technical solution of the present embodiment proposes that during the ablation hemostasis operation, the control system (or MCU controller) calculates impedance in real time based on collected data such as voltage and current. When the impedance meets preset conditions, it can be determined that the hemostasis ablation of the tissue at this location has met requirements, neither over-ablating and damaging the tissue nor failing to achieve hemostasis ablation. At this point, the biopsy needle is retracted by a certain distance to gradually perform hemostasis on the needle tract. Of course, in some solutions, due to the adjustable distance between the first electrode 001 and the second electrode 002 having different gear positions, after achieving hemostasis at one location, the electrodes do not completely exit the interval but retract by 1/2 (or other proportions) of the ablation interval for ablation, enabling flexible ablation hemostasis operations. The ablation interval here refers to the distance between the first electrode 001 and the second electrode 002.

The fully automatic biopsy excitation structure of the fully automatic biopsy needle with hemostasis function of the present invention is further described below.

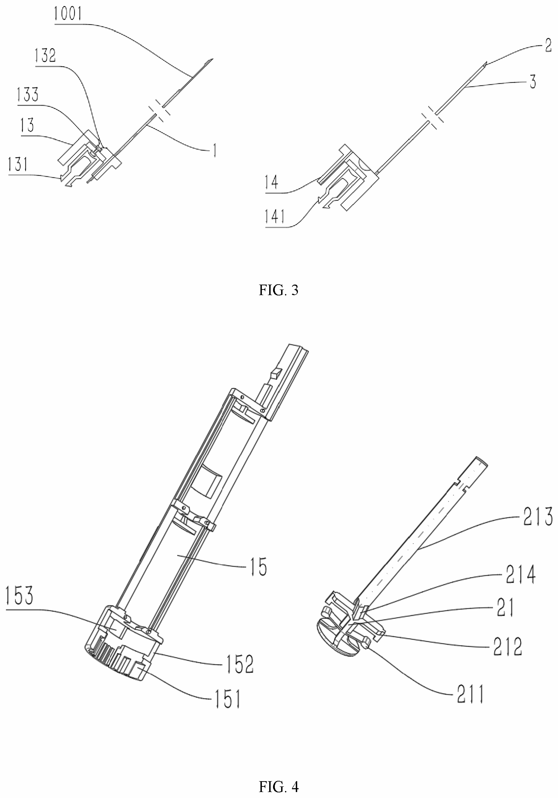

As shown in FIGS. 1, 2, 3, and 4, the needle body of the fully automatic biopsy needle includes a sampling needle 1, a cutting needle 2, and a cannula 4. The sampling needle 1 is fixedly connected to the sampling needle fixed slider 13, and the cutting needle 2 is fixedly connected to the cutting needle fixed slider 14. The sampling needle 1, the cutting needle 2, and the cannula 4 are coaxially nested, with an inner insulating layer 3 coaxially surrounding the cutting needle 2. The sampling needle fixed slider 13 is provided in the space formed by the left guide shell 15 and the right guide shell 16. The left guide shell 15 and the right guide shell 16 are provided with a first partition 17 that isolates the cutting needle fixed slider 14 from the sampling needle fixed slider 13. The first partition 17 has two functions: one is to act as a partition and limit, and the other is to facilitate the chambering of the cutting needle fixed slider 14. Specifically, a pair of first elastic clamping hooks 141 are provided at the bottom of the cutting needle fixed slider 14, and a first clamping groove 171 matching the first elastic clamping hooks 141 of the cutting needle fixed slider 14 is provided at the center of the first partition 17. The biopsy needle further includes a first spring 18, which is provided between the first partition 17 and the cutting needle fixed slider 14. When the cutting needle fixed slider 14 is pressed proximally for chambering, the first spring 18 compresses and deforms, driving the cutting needle 2 proximally until the first elastic clamping hooks 141 of the cutting needle fixed slider 14 pass through the first clamping groove 171, indicating that chambering is complete.

As shown in FIGS. 1,2,3, and 4, a pair of second elastic clamping hooks 131 are provided at the bottom of the sampling needle fixed slider 13. The left guide shell 15 and the right guide shell 16 are provided with a second partition 19, and a second clamping groove 191 matching the second elastic clamping hooks 131 of the sampling needle fixed slider 13 is provided at the center of the second partition 19. The biopsy needle further includes a second spring 20, which is provided between the second partition 19 and the sampling needle fixed slider 13. When the sampling needle fixed slider 13 is pressed proximally for chambering, the second spring 20 compresses and deforms, driving the sampling needle 1 proximally until the second elastic clamping hooks 131 of the sampling needle fixed slider 13 pass through the second clamping groove 191, indicating that chambering is complete. Here, I-shaped elastic gaskets 133 are provided on two sides of the sampling needle fixed slider 13 for cushioning. The sampling needle fixed slider 13 and the cutting needle fixed slider 14 have the same function and are basically the same in structure. The sampling needle fixed slider 13 is provided with a connector for the sampling needle button (No. 2 chambering button 12), and the cutting needle fixed slider 14 is provided with a connector for the cutting needle button (No. 1 chambering button 11).

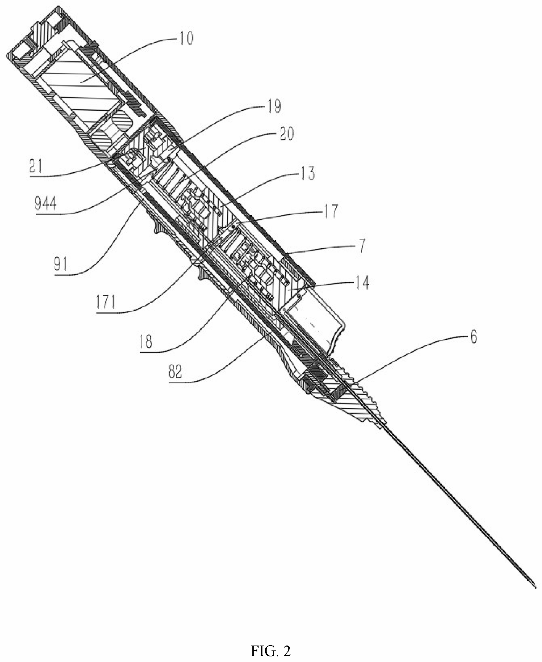

As shown in FIGS. 2, 4, 5, and 7, the fully automatic biopsy needle includes a firing switch 21. The body of the firing switch 21 is provided at the proximal end of the cavity formed by the left guide shell 15 and the right guide shell 16. The inner diameter of the cavity accommodating the firing switch 21 is larger than that accommodating the sampling needle fixed slider 13 and the cutting needle fixed slider 14, facilitating the extension of the linkage rod 213 of the firing switch 21 out of the left guide shell 15. The biopsy needle includes a shell of a handle 7 and a push button. the shell of the handle 7 is sleeved on the exterior of the left and right guide shells, and the push button is provided in the push button slot of the shell of the handle 7 and is engaged with the linkage rod 213. The firing switch 21 includes an elastic piece 211, a guiding limiting piece 212, and a linkage rod 213. The linkage rod 213 extends from the lower slot 153 of the left guide shell 15 to at least the upper edge of the upper opening. A limiting groove is provided on the exterior of the left guide shell 15 to limit the deflection of the linkage rod 213. A guiding limiting piece guide slot 152 is provided in the cavity accommodating the firing switch 21, and the guiding limiting piece 212 is provided in the guiding limiting piece guide slot 152 to ensure vertical movement within a limited stroke. An elastic piece limiting slot 151 is provided in the cavity accommodating the firing switch 21, and the elastic piece 211 is provided in the elastic piece limiting slot 151. The elastic piece 211 and the guiding limiting piece 212 are connected in the middle, with their free ends separated. Both ends of the elastic piece 211 abut against the inwardly extending internal abutment surfaces at the connection between the guiding limiting piece guide slot 152 and the elastic piece limiting slot 151. A first firing groove 214 is provided at the distal end of the guiding limiting piece 212 to compress the second elastic clamping hooks 131 of the sampling needle fixed slider 13 during firing. A second firing groove 132 is provided at the top of the sampling needle fixed slider 13 to compress the first elastic clamping hooks 141 of the cutting needle fixed slider 14 during firing.

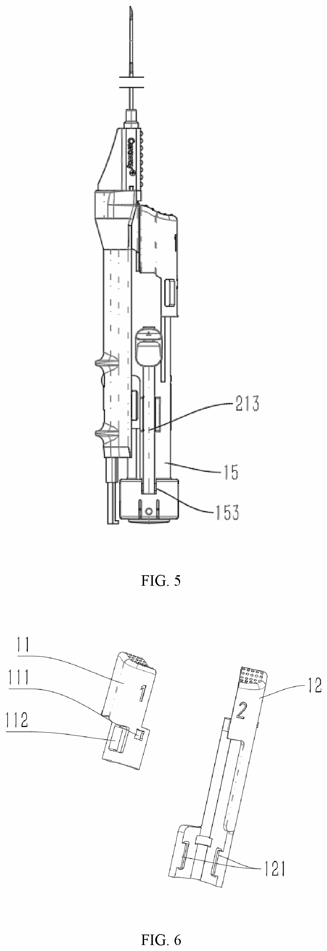

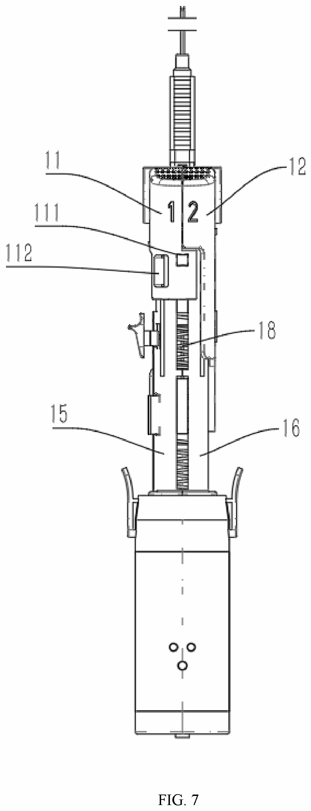

As shown in FIGS. 6 and 7, the biopsy needle further includes a No. 1 chambering button 11 and a No. 2 chambering button 12, which are matched in shape and connected. The No. 1 chambering button 11 is provided with a first connection hole 111, which is fixedly connected to the connector of the cutting needle button (No. 1 chambering button 11). The No. 1 chambering button 11 is inwardly provided with a first guiding plate 112, which is slidably connected to the first guiding groove provided on the upper exterior of the left guide shell 15 to ensure axial limiting movement. The No. 2 chambering button 12 is also provided with a second connection hole, which is fixedly connected to the connector of the sampling needle 1 button (No. 2 chambering button 12). The bottom of the No. 2 chambering button 12 is symmetrically provided with the second guiding plates 121, which are slidably connected to the second guiding groove provided at the lower end of the left guide shell 15 and the third guiding groove provided at the lower exterior of the right guide shell 16 to ensure vertical movement. Simultaneously, the second guiding plates 121 clamp the left guide shell 15 and the right guide shell 16.

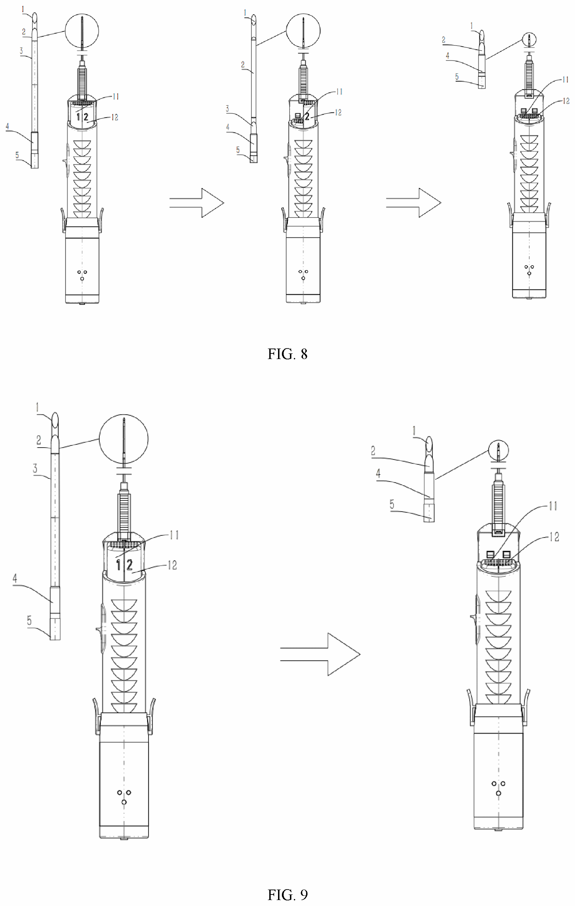

There are two methods for chambering:
Method 1: as shown in FIG. 8, the cutting needle 2 and the sampling needle 1 are performed chambering separately. The No. 1 chambering button 11 is pressed to drive the cutting needle fixed slider 14 to move the cutting needle 2 proximally to compress the first spring 18. When the first elastic clamping hooks 141 of the cutting needle fixed slider 14 pass through the first clamping groove 171, the cutting needle 2 is performed chambering. The No. 2 chambering button 12 drives the sampling needle fixed slider 13 to move the sampling needle 1 downward to compress the second spring 20. When the second elastic clamping hooks 131 of the sampling needle fixed slider 13 pass through the second clamping groove 191, the sampling needle 1 is performed chambering. At this point, chambering of the biopsy needle is separately completed.

Method 2: as shown in FIG. 9, the cutting needle 2 and the sampling needle 1 are performed chambering simultaneously. The No. 1 chambering button 11 and the No. 2 chambering button 12 are matched in shape and connected. The No. 2 chambering button 12 is compressed to simultaneously drive the No. 1 chambering button 11 to move, driving the cutting needle fixed slider 14 to move the cutting needle 2 downward, compressing the first spring 18. When the first elastic clamping hooks 141 of the cutting needle fixed slider 14 pass through the first clamping groove 171, the cutting needle 2 is performed chambering. Simultaneously, the sampling needle fixed slider 13 moves the sampling needle 1 downward, compressing the second spring 20. When the second elastic clamping hooks 131 of the sampling needle fixed slider 13 pass through the second clamping groove 191, the sampling needle 1 is performed chambering. At this point, the biopsy needle is performed chambering simultaneously.

### Biopsy excitation and sampling:

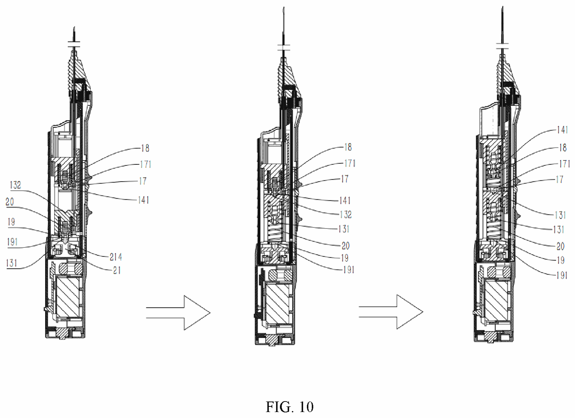

As shown in FIG. 10, after chambering is completed, the push button provided on the shell of the handle 7 or the button at the bottom of the biopsy device (firing switch 21) is pushed to drive the guiding limiting piece 212 to move toward the distal end. The first firing groove 214 abuts against the second elastic clamping hook 131; as the first firing groove 214 continuously presses the second elastic clamping hook 131, the second elastic clamping hook 131 is compressed and disengages from the second clamping groove 191, and the compressed second spring 20 is released to realize the firing of the sampling needle 1. Now when the firing ends, the sampling needle fixed slider 13 strikes the cutting needle fixed slider 14, causing the second firing groove 132 to strike the first elastic clamping hook 141 on the cutting needle fixed slider 14. During the instant strike process, the first elastic clamping hook 141 is compressed and disengages from the first clamping groove 171, and the compressed first spring 18 is released to realize the firing of the cutting needle 2. So far, the biopsy firing and sampling are completed.

### Embodiment 2

With the same concept, the present invention also provides a biopsy needle with hemostasis function, which includes:
a sampling needle 1 having a sampling groove 1001 provided at a distal end thereof;
a cutting needle assembly including a cutting needle 2 and an inner insulating layer 3 covering a periphery of the cutting needle 2, where a cutting edge that matches the sampling groove 1001 is provided at a distal end of the cutting needle 2, the cutting needle 2 is fitted over the periphery of the sampling needle 1, with the distal end of the cutting needle 2 exposed beyond an inner insulating layer 3 to form a first electrode 001 with the distal end of the sampling needle 1;
a cannula assembly including a cannula 4, an outer insulating layer 5, and a cannula plug 6, where the outer insulating layer 5 covers a periphery of a cannula 4, with a distal end of the cannula 4 exposed beyond the outer insulating layer 5 to form a second electrode 002, the cannula assembly is configured to be detachably connected to a handle 7 of a biopsy needle via the cannula plug 6, allowing the cannula 4 to be coaxially fitted over the periphery of the cutting needle 2 and be detachable;
the proximal end of the sampling needle 1 and the proximal end of the cannula plug 84 are respectively electrically connected to a power supply device 10 to disassemble the cannula assembly from the handle 7 after biopsy sampling, leaving the cannula assembly in a needle tract, and then insert the sampling needle 1 and the cutting needle assembly into the cannula after removing a sample tissue from the sampling groove 1001, and apply electrical energy to perform hemostasis operation on the needle tract 4 using the first electrode 001 and the second electrode 002.

According to the technical solution of the present embodiment, a cannula assembly is provided on the one hand to retain the needle tract and on the other hand to form two electrodes with the sampling needle 1 and the cutting needle 2 for needle tract ablation hemostasis. Specifically, after the sampling needle 1 and the cutting needle 2 complete sampling, they are withdrawn, leaving the cannula assembly in the needle tract to retain it. After removing the effectively sampled tissue, the sampling needle 1 and the cutting needle 2 are reinserted into the cannula 4, easily replicating the punctured needle tract during sampling. Moreover, the distal end of the cutting needle 2 forms a first electrode 001 with the distal end of the sampling needle 1, and the distal end of the cannula 4 forms a second electrode 002. By applying appropriate current to the first electrode 001 and the second electrode 002, tissue ablation and coagulation can be achieved, preventing needle tract bleeding. The biopsy needle in the present embodiment is not necessarily a fully automatic biopsy needle; it can be a semi-automatic or even a manual biopsy needle.

The present invention does not require two separate steps (or two separate products) to perform biopsy and ablation procedures, and does not require connection to a radiofrequency or microwave host during hemostasis. We integrate the functions required for biopsy and hemostasis from the bulky radiofrequency (microwave) host into disposable consumables, truly enabling the completion of both biopsy and hemostasis in a single step during outpatient surgery, which significantly reduces both surgical time and cost while greatly improving surgical safety. The present invention can be used for various tissue biopsies, especially for biopsy procedures with a high risk of bleeding, such as liver cancer and kidney cancer tumor biopsies, and has broad application prospects.

Furthermore, in the description of the present application, "proximal" and "distal" are commonly used terms in the medical field. Specifically, "proximal" refers to the end closer to the operator, "proximal end surface" refers to the end surface closer to the operator, "distal" refers to the end farther from the operator, and "distal end surface" refers to the end surface farther from the operator.

The above description of the embodiments of the present invention, in conjunction with the accompanying drawings, has been detailed. However, the present invention is not limited to the above embodiments. Even if various changes are made to the present invention, if these changes fall within the scope of the claims and their equivalents of the present invention, they are still considered to fall within the protection scope of the present invention.

## Claims

1. A biopsy needle with hemostasis function, comprising:
a sampling needle (1) having a sampling groove (1001) provided at a distal end thereof;
a cutting needle assembly comprising a cutting needle and an inner insulating layer (3) covering a periphery of the cutting needle, wherein a cutting edge that matches the sampling groove (1001) is provided at a distal end of the cutting needle, the cutting needle is fitted over a periphery of the sampling needle (1), with the distal end of the cutting needle exposed beyond the inner insulating layer (3) to form a first electrode (001) with the distal end of the sampling needle (1);
a cannula assembly comprising a cannula (4), an outer insulating layer (5), and a cannula plug (6), wherein the outer insulating layer (5) covers a periphery of a cannula (4), with a distal end of the cannula (4) exposed beyond the outer insulating layer (5) to form a second electrode, the cannula assembly is configured to be detachably connected to a handle (7) of the biopsy needle via the cannula plug (6), allowing the cannula (4) to be coaxially fitted over the periphery of the cutting needle and detachable; and
the proximal ends of the sampling needle (1) and the cannula plug (6) are respectively electrically connected to a power supply device (10), the cannula assembly being configured to be disassembled from the handle (7) after biopsy sampling, leaving the cannula assembly in a needle tract, the sampling needle (1) and the cutting needle assembly being configured to be inserted into the cannula (4) after removing a sample tissue from the sampling groove (1001), the power supply device (10) being configured to apply electrical energy to perform hemostasis operation on the needle tract using the first electrode (001) and the second electrode (002) after the the sampling needle (1) and the cutting needle assembly have been reinserted into the cannula (4).

2. The biopsy needle with hemostasis function according to claim 1, **characterized in that** the biopsy needle further comprises a distance adjusting block (8), which is slidably connected to the handle (7), with a distal end of the distance adjusting block (8) fixedly connected to the cannula plug (6), after inserting the sampling needle (1) and the cutting needle assembly into the cannula (4), the biopsy needle is configured to at least comprise a sampling configuration and a hemostasis configuration; in the sampling configuration, when the sampling needle (1) and the cutting needle complete sampling, a proximal end of the sampling groove (1001) extends beyond the distal end of the cannula (4); and in the hemostasis configuration, when the sampling needle (1) and the cutting needle maintain the completed sampling, the distance adjusting block (8) and the cannula assembly are adjusted a preset distance distally along a length of a needle body.

3. The biopsy needle with hemostasis function according to claim 2, **characterized in that** a cross-section of the distance adjusting block (8) matches that of the handle (7), with sliding buckles (81) provided on two sides of an inner wall of the distance adjusting block (8) extending along the length, an outer surface of the handle (7) is provided with chutes (71) that match the sliding buckles (81), the distance adjusting block (8) is configured to be slidably connected to the handle (7) via the sliding buckles (81) and the chutes (71) to enable distance adjustment along the length, after adjusting the distance adjusting block (8) to a predetermined position, a position of the distance adjusting block (8) is locked by a locking structure.

4. The biopsy needle with hemostasis function according to claim 3, **characterized in that** the locking structure comprises a plurality of locking holes (83) penetrating a surface of the distance adjusting block (8) and an elastic arm locking button (92) with one end fixedly arranged on the handle (7), a protrusion on a free end of the elastic arm locking button (92) is engaged with one of the locking holes (83) to lock axial relative positions of the first electrode (001) and the second electrode (002).

5. The biopsy needle with hemostasis function according to claim 2, **characterized in that** the biopsy needle further comprises an energy transmission socket (9), which is fixedly connected to the proximal end of the handle (7), with the distal end being a free end, an elastic arm locking button (92) for locking the axial relative positions of the first electrode (001) and the second electrode (002) is arranged on the free end of the energy transmission socket (9), and two electrical connection ends on the energy transmission socket (9) are respectively electrically connected to the cannula plug (6) and the proximal end of the sampling needle (1).

6. The biopsy needle with hemostasis function according to claim 5, **characterized in that** a first wiring groove (82) is provided at the inner wall of the distance adjusting block (8), and a second wiring groove (91) connected to the first wiring groove (82) is provided at an inner wall of the energy transmission socket (9), conducting wires connecting the cannula plug (6) to the energy transmission socket (9) and the sampling needle (1) to the energy transmission socket (9) extend along the first and/or second wiring grooves (91).

7. The biopsy needle with hemostasis function according to claim 5, **characterized by** further comprising a portable power supply device (10), which is electrically connected to the energy transmission socket (9) through an electrical pin, wherein the portable power supply device (10) comprises a lithium battery, a DCDC module, an MCU controller, an H-bridge module, and a step-up transformer, the lithium battery supplies power to the MCU controller through the DCDC module, the lithium battery is electrically connected to the H-bridge module, and the MCU controller outputs a driving signal to drive the H-bridge module to output current to the first electrode (001) and the second electrode (002) through the step-up transformer for tissue hemostasis.

8. The biopsy needle with hemostasis function according to claim 7, **characterized in that** the portable power supply device (10) is detachably connected to the proximal end of the handle (7), a first magnetic attraction piece (101) is provided inside a distal end of the portable power supply device (10), and a second magnetic attraction piece (72) is provided inside the proximal end of the handle (7), the distal end of the portable power supply device (10) is provided with elastic arms with convex clamping points on two sides of a circumference, and the proximal end of the handle (7) is provided with concave clamping points that match the convex clamping points.

9. The biopsy needle with hemostasis function according to claim 1, **characterized in that** the biopsy needle is configured to detect impedance in real-time via a control system during hemostasis operation, when the detected impedance meets a preset condition, the biopsy needle is retracted a preset distance and the hemostasis operation is repeated until the biopsy needle completely exits the needle tract.

10. The biopsy needle with hemostasis function according to any one of claim 1-9, **characterized in that**, the biopsy needle is a fully automatic biopsy needle, a semi-automatic biopsy needle or a manual biopsy needle.

## Patentansprüche

1. Biopsienadel mit Hämostasefunktion, umfassend:
eine Probennahmenadel (1), welche an einem distalen Ende davon eine Probennahmenut (1001) aufweist;
eine Schneidnadelbaugruppe, welche eine Schneidnadel und eine innere Isolierschicht (3) umfasst, welche einen Umfang der Schneidnadel abdeckt, wobei eine Schneidkante, welche der Probennahmenut (1001) entspricht, an einem distalen Ende der Schneidnadel bereitgestellt ist, die Schneidnadel über einen Umfang der Probennahmenadel (1) angebracht ist, wobei das distale Ende der Schneidnadel über die innere Isolierschicht (3) hinaus freiliegt, um eine erste Elektrode (001) mit dem distalen Ende der Probennahmenadel (1) zu bilden;
eine Kanülenbaugruppe, welche eine Kanüle (4), eine äußere Isolierschicht (5) und einen Kanülenstopfen (6) umfasst, wobei die äußere Isolierschicht (5) einen Umfang einer Kanüle (4) abdeckt, wobei ein distales Ende der Kanüle (4) über die äußere Isolierschicht (5) hinaus freiliegt, um eine zweite Elektrode zu bilden, wobei die Kanülenbaugruppe so konfiguriert ist, dass sie über den Kanülenstopfen (6) lösbar mit einem Griff (7) der Biopsienadel verbunden ist, was der Kanüle (4) erlaubt, koaxial über den Umfang der Schneidnadel angebracht und abnehmbar zu sein; und
die proximalen Enden der Probennahmenadel (1) und der Kanülenstopfen (6) mit einer Stromversorgungsvorrichtung (10) jeweils elektrisch verbunden sind, wobei die Kanülenbaugruppe so konfiguriert ist, dass sie nach der Biopsieentnahme vom Griff (7) demontiert wird, wobei die Kanülenbaugruppe in einem Nadelkanal verbleibt, wobei die Probennahmenadel (1) und die Schneidnadelbaugruppe so konfiguriert sind, dass sie nach dem Entfernen eines Probengewebes aus der Probennahmenut (1001) in die Kanüle (4) eingeführt werden, wobei die Stromversorgungsvorrichtung (10) dazu konfiguriert ist, elektrische Energie anzuwenden, um einen Hämostasevorgang am Nadeltrakt unter Verwendung der ersten Elektrode (001) und der zweiten Elektrode (002) durchzuführen, nachdem die Probennahmenadel (1) und die Schneidnadelbaugruppe wieder in die Kanüle (4) eingeführt wurden.

2. Biopsienadel mit Hämostasefunktion nach Anspruch 1, **dadurch gekennzeichnet, dass** die Biopsienadel weiter einen Abstandseinstellblock (8) umfasst, welcher mit dem Griff (7) verschiebbar verbunden ist, wobei ein distales Ende des Abstandseinstellblocks (8) fest mit dem Kanülenstopfen (6) verbunden ist, nachdem die Probenahmenadel (1) und die Schneidnadelbaugruppe in die Kanüle (4) eingeführt wurden, die Biopsienadel so konfiguriert ist, dass sie zumindest eine Probenahmekonfiguration und eine Hämostasekonfiguration umfasst; wenn in der Probennahmekonfiguration die Probennahmenadel (1) und die Schneidnadel die Probennahme abschließen, ein proximales Ende der Probennahmenut (1001) über das distale Ende der Kanüle (4) hinausragt; und wenn in der Hämostasekonfiguration die Probennahmenadel (1) und die Schneidnadel die abgeschlossene Probennahme aufrechterhalten, der Abstandseinstellblock (8) und die Kanülenbaugruppe um einen voreingestellten Abstand distal entlang einer Länge eines Nadelkörpers eingestellt werden.

3. Biopsienadel mit Hämostasefunktion nach Anspruch 2, **dadurch gekennzeichnet, dass** ein Querschnitt des Abstandseinstellblocks (8) dem des Griffs (7) entspricht, wobei an zwei Seiten einer sich entlang der Länge erstreckenden Innenwand des Abstandseinstellblocks (8) Schiebeschnallen (81) bereitgestellt sind, eine Außenfläche des Griffs (7) Rinnen (71) umfasst, welche den Schiebeschnallen (81) entsprechen, wobei der Abstandseinstellblock (8) so konfiguriert ist, dass er mit dem Griff (7) über die Schiebeschnallen (81) und die Rinnen (71) verschiebbar verbunden ist, um eine Abstandsverstellung in Längsrichtung zu ermöglichen, nach Einstellen des Abstandseinstellblocks (8) auf eine vorbestimmte Position, eine Position des Abstandseinstellblocks (8) durch eine Verriegelungsstruktur verriegelt wird.

4. Biopsienadel mit Hämostasefunktion nach Anspruch 3, **dadurch gekennzeichnet, dass** die Verriegelungsstruktur eine Vielzahl von Verriegelungslöchern (83), welche eine Oberfläche des Abstandseinstellblocks (8) durchdringen, und einen elastischen Armverriegelungsknopf (92) mit einem Ende umfasst, welches fest am Griff (7) angeordnet ist, ein Vorsprung an einem freien Ende des elastischen Armverriegelungsknopfes (92) in eines der Verriegelungslöcher (83) eingreift, um axiale relative Positionen der ersten Elektrode (001) und der zweiten Elektrode (002) zu verriegeln.

5. Biopsienadel mit Hämostasefunktion nach Anspruch 2, **dadurch gekennzeichnet, dass** die Biopsienadel weiter eine Energieübertragungsbuchse (9) umfasst, welche fest mit dem proximalen Ende des Griffs (7) verbunden ist, wobei das distale Ende ein freies Ende ist, eine elastische Armarretiertaste (92) zum Arretieren der axialen relativen Positionen der ersten Elektrode (001) und der zweiten Elektrode (002) am freien Ende der Energieübertragungsbuchse (9) angeordnet ist, und zwei elektrische Anschlussenden an der Energieübertragungsbuchse (9) jeweils elektrisch mit dem Kanülenstopfen (6) und dem proximalen Ende der Probennahmenadel (1) verbunden sind.

6. Biopsienadel mit Hämostasefunktion nach Anspruch 5, **dadurch gekennzeichnet, dass** eine erste Verdrahtungsnut (82) an der Innenwand des Abstandseinstellblocks (8) bereitgestellt ist, und eine zweite mit der ersten Verdrahtungsnut (82) verbundene Verdrahtungsnut (91) an einer Innenwand der Energieübertragungsbuchse (9) bereitgestellt ist, Leiterdrähte, welche den Kanülenstecker (6) mit der Energieübertragungsbuchse (9) und die Probennahmenadel (1) mit der Energieübertragungsbuchse (9) verbinden, sich entlang der ersten und/oder zweiten Kabelführungsnut (91) erstrecken.

7. Biopsienadel mit Hämostasefunktion nach Anspruch 5, **dadurch gekennzeichnet, dass** sie weiter eine tragbare Stromversorgungsvorrichtung (10) umfasst, welche über einen elektrischen Stift elektrisch mit der Energieübertragungsbuchse (9) verbunden ist, wobei die tragbare Stromversorgungsvorrichtung (10) eine Lithiumbatterie, ein DCDC-Modul, eine MCU-Steuerung, ein H-Brückenmodul und einen Aufwärtstransformator umfasst, die Lithiumbatterie die MCU-Steuerung über das DCDC-Modul mit Strom versorgt, die Lithiumbatterie mit dem H-Brückenmodul elektrisch verbunden ist, und die MCU-Steuerung ein Antriebssignal ausgibt, um das H-Brückenmodul anzusteuern, um Strom an die erste Elektrode (001) und die zweite Elektrode (002) durch den Aufwärtstransformator für die Gewebehämostase auszugeben.

8. Biopsienadel mit Hämostasefunktion nach Anspruch 7, **dadurch gekennzeichnet, dass** die tragbare Stromversorgungseinrichtung (10) lösbar mit dem proximalen Ende des Griffs (7) verbunden ist, ein erstes magnetisches Anziehungsstück (101) innerhalb eines distalen Endes der tragbaren Stromversorgungseinrichtung (10) bereitgestellt ist, und ein zweites magnetisches Anziehungsstück (72) innerhalb des proximalen Endes des Griffs (7) bereitgestellt ist, das distale Ende der tragbaren Stromversorgungseinrichtung (10) elastische Arme mit konvexen Klemmpunkten auf zwei Seiten eines Umfangs umfasst, und das proximale Ende des Griffs (7) konkave Klemmpunkte umfasst, welche den konvexen Klemmpunkten entsprechen.

9. Biopsienadel mit Hämostasefunktion nach Anspruch 1, **dadurch gekennzeichnet, dass** die Biopsienadel so konfiguriert ist, dass sie während des Hämostasevorgangs über ein Kontrollsystem Impedanz in Echtzeit erkennt, wenn die erkannte Impedanz eine voreingestellte Bedingung erfüllt, die Biopsienadel um einen voreingestellten Abstand zurückgezogen wird und der Hämostasevorgang wiederholt wird, bis die Biopsienadel den Nadeltrakt vollständig verlässt.

10. Biopsienadel mit Hämostasefunktion nach einem der Ansprüche 1 bis 9, **dadurch gekennzeichnet, dass** die Biopsienadel eine vollautomatische Biopsienadel, eine halbautomatische Biopsienadel oder eine manuelle Biopsienadel ist.

## Revendications

1. Aiguille de biopsie à fonction d'hémostase, comprenant :
une aiguille de prélèvement (1) présentant une rainure de prélèvement (1001) disposée à une extrémité distale de celle-ci ;
un ensemble aiguille tranchante comprenant une aiguille tranchante et une couche isolante interne (3) recouvrant une périphérie de l'aiguille tranchante, dans laquelle un bord de coupe qui correspond à la rainure de prélèvement (1001) est disposé à une extrémité distale de l'aiguille tranchante, l'aiguille tranchante est montée sur une périphérie de l'aiguille de prélèvement (1), l'extrémité distale de l'aiguille tranchante étant exposée au-delà de la couche isolante interne (3) pour former une première électrode (001) avec l'extrémité distale de l'aiguille de prélèvement (1) ;
un ensemble canule comprenant une canule (4), une couche isolante externe (5) et un bouchon de canule (6), dans laquelle la couche isolante externe (5) recouvre une périphérie d'une canule (4), une extrémité distale de la canule (4) étant exposée au-delà de la couche isolante externe (5) pour former une deuxième électrode, l'ensemble canule est destiné à être relié de manière détachable à un manche (7) de l'aiguille de biopsie par l'intermédiaire du bouchon de canule (6), permettant le montage coaxial et détachable de la canule (4) sur la périphérie de l'aiguille tranchante ; et
les extrémités proximales de l'aiguille de prélèvement (1) et du bouchon de canule (6) sont respectivement connectées électriquement à un dispositif d'alimentation électrique (10), l'ensemble canule étant destiné à être démonté du manche (7) après prélèvement par biopsie, laissant l'ensemble canule dans un trajet d'aiguille, l'aiguille de prélèvement (1) et l'ensemble aiguille tranchante étant destinés à être insérés dans la canule (4) après retrait d'un prélèvement de tissu de la rainure de prélèvement (1001), le dispositif d'alimentation électrique (10) étant destiné à appliquer de l'énergie électrique pour effectuer une opération d'hémostase sur le trajet d'aiguille à l'aide de la première électrode (001) et de la deuxième électrode (002) après que l'aiguille de prélèvement (1) et l'ensemble aiguille tranchante ont été réinsérés dans la canule (4).

2. Aiguille de biopsie à fonction d'hémostase selon la revendication 1, **caractérisée en ce que** l'aiguille de biopsie comprend en outre un bloc de réglage de distance (8), qui est relié de manière coulissante au manche (7), une extrémité distale du bloc de réglage de distance (8) étant reliée à demeure au bouchon de canule (6), après insertion de l'aiguille de prélèvement (1) et de l'ensemble aiguille tranchante dans la canule (4), l'aiguille de biopsie est destinée à comprendre au moins une configuration de prélèvement et une configuration d'hémostase ; dans la configuration de prélèvement, lorsque l'aiguille de prélèvement (1) et l'aiguille tranchante réalisent le prélèvement, une extrémité proximale de la rainure de prélèvement (1001) s'étend au-delà de l'extrémité distale de la canule (4) ; et dans la configuration d'hémostase, lorsque l'aiguille de prélèvement (1) et l'aiguille tranchante maintiennent le prélèvement réalisé, le bloc de réglage de distance (8) et l'ensemble canule sont réglés à une distance prédéfinie de manière distale sur une longueur d'un corps d'aiguille.

3. Aiguille de biopsie à fonction d'hémostase selon la revendication 2, **caractérisée en ce qu'**une section transversale du bloc de réglage de distance (8) correspond à celle du manche (7), avec des attaches coulissantes (81) disposées sur deux côtés d'une paroi interne du bloc de réglage de distance (8) s'étendant sur la longueur, une surface externe du manche (7) est munie de goulottes (71) qui correspondent aux attaches coulissantes (81), le bloc de réglage de distance (8) est destiné à être relié de manière coulissante au manche (7) par l'intermédiaire des attaches coulissantes (81) et des goulottes (71) pour permettre un réglage de distance sur la longueur, après réglage du bloc de réglage de distance (8) sur une position prédéterminée, une position du bloc de réglage de distance (8) est verrouillée par une structure de verrouillage.

4. Aiguille de biopsie à fonction d'hémostase selon la revendication 3, **caractérisée en ce que** la structure de verrouillage comprend une pluralité de trous de verrouillage (83) pénétrant une surface du bloc de réglage de distance (8) et un bouton de verrouillage (92) à bras élastique dont une extrémité est agencée à demeure sur le manche (7), une protubérance sur une extrémité libre du bouton de verrouillage (92) à bras élastique est en prise avec l'un des trous de verrouillage (83) pour verrouiller des positions axiales relatives de la première électrode (001) et de la deuxième électrode (002).

5. Aiguille de biopsie à fonction d'hémostase selon la revendication 2, **caractérisée en ce que** l'aiguille de biopsie comprend en outre une douille de transmission d'énergie (9), qui est reliée à demeure à l'extrémité proximale du manche (7), l'extrémité distale étant une extrémité libre, un bouton de verrouillage (92) à bras élastique pour verrouiller les positions relatives axiales de la première électrode (001) et de la deuxième électrode (002) est agencé sur l'extrémité libre de la douille de transmission d'énergie (9), et deux extrémités de connexion électrique sur la douille de transmission d'énergie (9) sont respectivement connectées électriquement au bouchon de canule (6) et à l'extrémité proximale de l'aiguille de prélèvement (1).

6. Aiguille de biopsie à fonction d'hémostase selon la revendication 5, **caractérisée en ce qu'**une première rainure de câblage (82) est disposée au niveau de la paroi interne du bloc de réglage de distance (8), et une deuxième rainure de câblage (91) reliée à la première rainure de câblage (82) est disposée au niveau d'une paroi interne de la douille de transmission d'énergie (9), des fils conducteurs reliant le bouchon de canule (6) à la douille de transmission d'énergie (9) et l'aiguille de prélèvement (1) à la douille de transmission d'énergie (9) s'étendent le long des première et/ou deuxième rainures de câblage (91).

7. Aiguille de biopsie à fonction d'hémostase selon la revendication 5, **caractérisée en outre en ce qu'**elle comprend un dispositif d'alimentation électrique portable (10), qui est connecté électriquement à la douille de transmission d'énergie (9) par l'intermédiaire d'une broche électrique, dans laquelle le dispositif d'alimentation électrique portable (10) comprend une batterie au lithium, un module CC-CC, un contrôleur MCU, un module pont en H et un transformateur élévateur, la batterie au lithium alimente en énergie le contrôleur MCU par l'intermédiaire du module CC-CC, la batterie au lithium est connectée électriquement au module pont en H et le contrôleur MCU émet un signal de pilotage pour piloter le module pont en H à émettre un courant vers la première électrode (001) et la deuxième électrode (002) à travers le transformateur élévateur pour l'hémostase tissulaire.

8. Aiguille de biopsie à fonction d'hémostase selon la revendication 7, **caractérisée en ce que** le dispositif d'alimentation électrique portable (10) est connecté de manière détachable à l'extrémité proximale du manche (7), une première partie d'attraction magnétique (101) est disposée à l'intérieur d'une extrémité distale du dispositif d'alimentation électrique portable (10), et une deuxième partie d'attraction magnétique (72) est disposée à l'intérieur de l'extrémité proximale du manche (7), l'extrémité distale du dispositif d'alimentation électrique portable (10) est munie de bras élastiques avec des points de serrage convexes sur deux côtés d'une circonférence, et l'extrémité proximale du manche (7) est munie de points de serrage concaves qui correspondent aux points de serrage convexes.

9. Aiguille de biopsie à fonction d'hémostase selon la revendication 1, **caractérisée en ce que** l'aiguille de biopsie est destinée à détecter l'impédance en temps réel par l'intermédiaire d'un système de commande pendant l'opération d'hémostase, lorsque l'impédance détectée répond à une condition prédéfinie, l'aiguille de biopsie est rétractée d'une distance prédéfinie et l'opération d'hémostase est répétée jusqu'à ce que l'aiguille de biopsie sorte complètement du trajet d'aiguille.

10. Aiguille de biopsie à fonction d'hémostase selon l'une quelconque des revendications 1-9, **caractérisée en ce que** l'aiguille de biopsie est une aiguille de biopsie entièrement automatique, une aiguille de biopsie semi-automatique ou une aiguille de biopsie manuelle.
